# EUROPÄISCHE PATENTANMELDUNG

(11) **EP 4 818 693 A1**
(43) Veröffentlichungstag der Anmeldung: **07.10.2026**
(21) Anmeldenummer: 26167585.4
(22) Anmeldetag: 25.03.2026
(51) Int. Cl.: G01N 29/032, G01N 29/32, G01N 29/34

(54) **DETEKTOR ZUR ERKENNUNG VON EINSCHLÜSSEN IN FLÜSSIGKEIT, MEDIZINISCHES BEHANDLUNGSGERÄT DAMIT, UND VERFAHREN ZUR ERKENNUNG VON EINSCHLÜSSEN IN FLÜSSIGKEIT**

(30) Priorität: 01.04.2025 DE 102025112777
(71) Anmelder: B. Braun Avitum AG, 34212 Melsungen (DE)
(72) Erfinder: HOLLSTEIN, Rolf, 36211 Alheim-Heinebach (DE); KRAPF, Johannes, 36179 Bebra (DE); BRESSAU, Ernst, 34323 Malsfeld (DE)
(74) Vertreter: Winter, Brandl - Partnerschaft mbB

(57) **Zusammenfassung**

Offenbart ist ein Detektor (1) zur Erkennung von Einschlüssen in einer extrakorporal strömenden Flüssigkeit auf Basis von Ultraschall mit einem Ultraschall-Sender (4), einem Ultraschall-Empfänger (8) und einer Steuereinheit (30), wobei die Steuereinheit (30) ausgestaltet ist, den Ultraschall-Sender (4) mit einem Ansteuersignal (18) anzusteuern, das eine Abfolge von Impulsen (L, S) umfasst, wobei die Abfolge einen Ladeimpuls (L) mit einer Ladezeit (B) und eine, auf den Ladeimpuls (L) folgende, Anzahl (C) von Erregungsimpulsen (S) umfasst, der Ultraschall-Sender (4) ausgestaltet ist, einen Ultraschall (6) mit einer dem Ansteuersignal (18) gemäßen Sendeenergie durch einen die Flüssigkeit führenden Kanal (2) zu senden, der Ultraschall-Empfänger (8) ausgestaltet ist, ein einer Empfangsenergie des Ultraschalls (6') gemäßes Empfangssignal (20) bereitzustellen, und die Steuereinheit (30) ausgestaltet ist, in Abhängigkeit des Empfangssignals (20) die Sendeenergie anzupassen, um das Empfangssignal (20), vorzugsweise ein vom Empfangssignal (20) abgeleitetes Signal (tm), auf einen Zielwert (ts) oder in ein Zielwert-Intervall (tsmin, tsmax) zu führen, wobei die Steuereinheit (30) ausgestaltet ist, die Anzahl (C) von Erregungsimpulsen (S) und/ oder die Ladezeit (B) des Ladeimpulses (L) anzupassen, um das Empfangssignal (20), vorzugsweise das vom Empfangssignal (20) abgeleitete Signal (tm), auf den Zielwert (ts) oder in das Zielwertintervall (tsmin, tsmax) zu führen. Offenbart ist weiterhin ein medizinisches Behandlungsgerät mit einem Detektor, ein computerimplementiertes Verfahren für einen Detektor und ein computerlesbares Speichermedium.

## Beschreibung

### Technisches Gebiet

Die vorliegende Offenbarung betrifft einen Detektor auf Ultraschallbasis zur Erkennung von Einschlüssen in strömender Flüssigkeit, ein medizinisches Behandlungsgerät damit, ein computerimplementiertes Verfahren zur Erkennung von Einschlüssen in strömender Flüssigkeit auf Ultraschallbasis, sowie ein computerlesbares Speichermedium.

In medizinischen Behandlungsgeräten, insbesondere Dialysegeräten, werden üblicherweise Detektoren auf Ultraschallbasis (im Folgenden als Detektor bezeichnet) eingesetzt, um Patienten vor gefährlichen Luftembolien zu schützen. Hierfür kann ein zu überwachender Teil eines extrakorporalen Blutkreislaufes in Form eines Blutschlauchs durch eine Ultraschallstrecke des Detektors geführt werden. Der Detektor hat hierzu ein sendendes Piezoelement, das durch elektrische Anregung auf seine Resonanzfrequenz einen Ultraschall-Impuls, bzw. Ultraschall-Wellen durch den Blutschlauch sendet. Den Ultraschall-Impuls kann ein empfangendes Piezoelement des Detektors empfangen und in ein Empfangssignal, bzw. eine Empfangs-Spannung wandeln. Dieses, bzw. diese wird von einem Komparator mit einer Referenzspannung verglichen und bewertet. Ausgangsseitig des Komparators steht so ein vom Empfangssignal abgeleitetes Signal zur Verfügung, welches Auskunft darüber gibt, ob sich in der Ultraschallstrecke "Luft" oder "keine Luft" (also nur Flüssigkeit bzw. Blut) befindet.

Toleranzen entlang der Ultraschallstrecke führen zu Toleranzen in der Kopplung und in der Empfindlichkeit des Detektors gegenüber Luftblasen. Zu nennen sind insbesondere Toleranzen in Piezoschichten und im Verguss der Piezoelemente, im Gehäusematerial oder in der Geometrie des Detektors, sowie im Material, in der Geometrie, im Herstellungsverfahren oder im Sterilisationsverfahren des als Einmalprodukt verwendeten Schlauchs.

Ein Arbeitsbereich des Detektors zwischen der "Verfügbarkeit" eines ausreichend starken Empfangssignals, das eine sichere Detektion ermöglicht, und der "Sicherheit", die zu überwachende Flüssigkeit/ das Blut nicht durch zu hohen Energieeintrag zu gefährden, ist aufgrund der genannten Toleranzen vergleichsweise eng.

Mit dem Aspekt der Sicherheit befasst sich die Veröffentlichung "Bildgebende Verfahren in der Medizin. Von der Technik zur medizinischen Anwendung." von Prof. Dr. Olaf Dössel, Univ. Karlsruhe, ISBN 3-540-66014-3. Dargestellt wird, dass die Ultraschall-Impulse des Detektors das durch den Blutschlauch geführte Blut des Patienten bei einem zu hohen Energieeintrag schädigen. Die Schädigung erfolgt hierbei insbesondere durch Wärmeeinwirkung und durch Kavitation. Wärme entsteht lokal proportional zur vom Blut absorbierten Schallintensität. Bei der Kavitation handelt es sich um einen Effekt, bei dem in einer Unterdruckphase eines Ultraschall-Impulses im Gewebe Gasbläschen entstehen, die dann in einer Druckphase kollabieren.

Eine Anforderung ist somit, die Sicherheit des Detektors zu gewährleisten, indem der mit dem Ultraschall verbundene Energieeintrag überwacht und begrenzt wird. Hierzu schlägt die Druckschrift EP 3 070 466 A1 vor, den Energieeintrag mittelbar anhand des Ansteuer-Signals zu überwachen, mit dem der Ultraschall-Sender beaufschlagt, bzw. angeregt wird. Im Fehlerfall, wenn die Überwachung ergibt, dass sich das AnsteuerSignal so verändert, dass der daraus resultierende Energieeintrag ein verträgliches Maß überschreitet, werden Hardware-Maßnahmen hydraulischer und/ oder elektrischer/ elektronischer Natur ergriffen. Beispielsweise können Pumpen gestoppt oder Ventile geschlossen werden, um den Volumenstrom des Mediums/ Blutes zu unterbrechen. Elektrisch/ elektronische Maßnahmen zielen auf die Abschaltung oder Begrenzung des Energieeintrags ab. Beispielsweise kann die Energiezufuhr zum Ultraschall-Sender unterbrochen werden. Alternativ oder ergänzend kann ein elektrisches oder elektronisches Bauteil so dimensioniert oder ausgewählt sein, dass darüber der Energieeintrag begrenzt werden kann - beispielsweise indem eine Dimensionierung, bzw. eine Auswahl einer Induktivität mit einem entsprechenden Sättigungsstrom vorgesehen ist oder Z-Dioden zur Spannungsbegrenzung vorgesehen sind.

Wie oben bereits erwähnt, ist eine weitere Anforderung die Verfügbarkeit des Detektors, insbesondere eine ausreichende Signalstärke des Empfangssignals, zu gewährleisten. Die Druckschrift EP 0 643 301 B1 schlägt hierzu vor, einen von einer Sequenz des Empfangssignals abgeleiteten Mittelwert gegen ein vorbestimmtes Soll abzugleichen und in Abhängigkeit der Abweichung eine Amplitude des Ansteuersignals, mit dem der Ultraschall-Sender beaufschlagt wird, anzupassen. So werden Toleranzen und Alterungseffekte der Ultraschallstrecke kompensiert und der Mittelwert des Empfangssignals kann auf einem geforderten Niveau konstant gehalten werden.

Demgegenüber liegt der Erfindung die Aufgabe zugrunde, einen Detektor von Einschlüssen in strömender Flüssigkeit auf Ultraschallbasis zu schaffen, mit dem Toleranzen und Alterungseffekte seiner Ultraschallstrecke flexibel zu kompensieren sind. Des Weiteren liegt der Erfindung die Aufgabe zugrunde, ein medizinisches Behandlungsgerät, ein computerimplementiertes Verfahren und ein computerlesbares Speichermedium zu schaffen, mit dem Toleranzen und Alterungseffekte einer Ultraschallstrecke flexibel zu kompensieren sind.

Die jeweilige Aufgabe wird gelöst durch einen Detektor mit den Merkmalen gemäß Anspruch 1, durch ein medizinisches Behandlungsgerät mit den Merkmalen gemäß Anspruch 8, durch ein Verfahren mit den Merkmalen gemäß Anspruch 9 und durch ein computerlesbares Speichermedium gemäß Anspruch 15. Sonstige vorteilhafte Weiterbildungen der Offenbarung sind Gegenstand der Unteransprüche und/oder werden nachfolgend erläutert.

Ein Grundgedanke der Offenbarung besteht darin, ein Empfangssignal einer Ultraschallstrecke zu erfassen, das Empfangssignal oder ein davon abgeleitetes Signal gegen einen Zielwert oder ein Zielwert-Intervall auszuwerten/ abzugleichen und in Abhängigkeit der Auswertung/ des Abgleichs ein Ansteuersignal, von dem eine Sendeenergie des Ultraschalls abhängt, anzupassen, um so das Empfangssignal oder das abgeleitete Signal auf den Zielwert oder in das Zielwert-Intervall zu führen. Das Ansteuersignal ist zeitlich strukturiert und umfasst eine zeitliche Abfolge von Impulsen. Offenbarungsgemäß erfolgt die Anpassung, indem die zeitliche Abfolge der Impulse angepasst wird, und zwar bezüglich einer Dauer, Ladedauer oder Ladezeit eines Ladeimpulses und/ oder einer Anzahl von Erregungsimpulsen. Offenbarungsgemäß können so Toleranzen und Alterungseffekte der Ultraschallstrecke des Detektors, insbesondere Veränderungen in der Kopplung oder Dämpfung der Ultraschallstrecke, flexibel kompensiert werden. Das heißt, es wird ein Empfangssignal oder abgeleitetes Signal bereitgestellt, dessen Signalstärke wiederholsicher und verlässlich ist. Die Qualität des Empfangssignals oder abgeleiteten Signals ist somit erhöht.

Die Qualität umfasst insbesondere, dass das Empfangssignal oder abgeleitete Signal in einem optimalen Arbeitsbereich der Signal-Auswertung bereitgestellt ist, und/ oder dass ein akzeptables Signal-Rausch-Verhältnis (Signal to Noise Ratio, SNR) bereitgestellt ist, welches eine zuverlässige Signalanalyse ermöglicht.

Ein offenbarungsgemäßer Detektor ist zur Erkennung von Einschlüssen in einer extrakorporal strömenden Flüssigkeit auf Basis von Ultraschall vorgesehen. Insbesondere ist er zur Erkennung von Blasen in Blut oder in medizinischer Lösung, insbesondere für ein Blutbehandlungsgerät, insbesondere Dialysegerät, bzw. für eine Infusionspumpe, vorgesehen. Der Detektor hat einen Ultraschall-Sender, einen Ultraschall-Empfänger und eine Steuereinheit, wobei:
- die Steuereinheit ausgestaltet ist, den Ultraschall-Sender mit einem Ansteuersignal anzusteuern, das eine Abfolge von Impulsen umfasst, wobei die Abfolge einen Ladeimpuls mit einer Ladedauer oder Ladezeit und eine auf den Ladeimpuls folgende Anzahl von Erregungsimpulsen umfasst,
- der Ultraschall-Sender ausgestaltet ist, einen Ultraschall mit einer dem Ansteuersignal gemäßen Sendeenergie durch einen Kanal zu senden, der vorgesehen ist, die Flüssigkeit zu führen,
- der Ultraschall-Empfänger ausgestaltet ist, den Ultraschall zu empfangen und ein Empfangssignal bereitzustellen, dass einer Empfangsenergie des Ultraschalls entspricht, und
- die Steuereinheit ausgestaltet ist, in Abhängigkeit des Empfangssignals die Sendeenergie anzupassen, um das Empfangssignal oder ein davon abgeleitetes Signal auf einen Zielwert oder in ein Zielwert-Intervall zu führen und somit ein Empfangssignal oder abgeleitetes Signal zu erhalten, dessen Signalstärke wiederholsicher und verlässlich ist. Die Qualität des Empfangssignals oder abgeleiteten Signals ist somit erhöht. Offenbarungsgemäß ist die Steuereinheit ausgestaltet, innerhalb der Abfolge des Ansteuersignals die Anzahl von Erregungsimpulsen oder die Ladezeit des Ladeimpulses, oder beide, anzupassen, um dadurch das Empfangssignal oder das abgeleitete Signal auf den Zielwert oder in das Zielwertintervall zu führen.

So ist ein Detektor geschaffen, bei dem Toleranzen und Alterungseffekte seiner Ultraschallstrecke sehr flexibel zu kompensieren sind:
- durch Anpassung der Ladezeit des Ladeimpulses der Abfolge,
- durch Anpassung der Anzahl von Erregungsimpulsen der Abfolge, oder
- durch Anpassung sowohl der Ladezeit des Ladeimpulses als auch der Anzahl von Erregungsimpulsen.

Ergänzend kann die Anpassung durch eine Anpassung einer Amplitude der Ultraschall-Sende-Impulse erfolgen.

Gemäß einer Weiterbildung ist die Steuereinheit ausgestaltet, das Empfangssignal mit einem Referenzwert zu vergleichen und in Abhängigkeit des Vergleichs ein Ausgabesignal auszugeben, das ein Detektions- oder Erkennungsergebnis bezüglich von Einschlüssen anzeigt, vorzugsweise quantifiziert.

Gemäß einer Weiterbildung sind der Ultraschall-Sender und der Ultraschall-Empfänger jeweils von einem Piezoelement gebildet und elektrisch parallel zum Ultraschall-Sender ist eine Induktivität als Spannungseinrichtung vorgesehen.

Die offenbarungsgemäße Abfolge von Impulsen des Ansteuersignals umfasst den Ladeimpuls mit der Ladedauer oder Ladezeit, während der die Induktivität mit einer Spannungsquelle verbunden ist und aufgeladen wird.

In der Abfolge von Impulsen folgt auf den Ladeimpuls offenbarungsgemäß die (Impuls-) Anzahl von Erregungsimpulsen, um den Ultraschall-Sender anzuregen.

Vorzugsweise definiert die Abfolge von Impulsen und eine darauffolgende Pause ein Burst-Intervall des Ansteuersignals. Das Burst-Intervall definiert somit die Zeitspanne zwischen Startzeitpunkten von aufeinanderfolgenden Ladeimpulsen, bzw. des Ansteuersignals.

Vorzugsweise ist die Steuereinheit ausgestaltet, den Ultraschall-Sender mit einer Sequenz von Ansteuersignalen, bzw. Burst-Intervallen, anzusteuern. Daraus resultiert eine entsprechende Sequenz von gesendeten Ultraschallsignalen und eine entsprechende Sequenz von Empfangssignalen.

Vorzugsweise ist das vom Empfangssignal abgeleitete Signal ein statistischer Wert oder eine statistische Größe, insbesondere ein Median, ein Mittelwert, ein Maximalwert und/ oder eine Standardabweichung der Sequenz von Empfangssignalen.

Gemäß einer Weiterbildung umfasst die Steuereinheit eine Korrelation der Anzahl von Erregungsimpulsen und/ oder der Ladezeit des Ladeimpulses, und insbesondere der daraus resultierenden Sendeenergie, in Abhängigkeit wenigstens eines Parameters des Detektors, des Kanals und/ oder der Flüssigkeit. Insbesondere umfasst die Korrelation eine Look-up-Tabelle und/ oder einen Algorithmus. Mittels der Korrelation umfasst die Steuereinheit offenbarungsgemäß geeignete Initialwerte oder Startwerte für die Anzahl von Erregungsimpulsen und/ oder die Ladezeit des Ladeimpulses, mit denen das Empfangssignal oder das davon abgeleitete Signal initial bereits möglichst nahe an den Zielwert oder an (in) das Zielwert-Intervall geführt werden kann und somit nachfolgende Anpassungen der Anzahl von Erregungsimpulsen und/ oder der Ladezeit des Ladeimpulses minimiert sind oder sogar entfallen können.

Vorzugsweise ist die Steuereinheit ausgestaltet, in Kenntnis des wenigstens einen Parameters, beispielsweise nach dessen Eingabe, Erfassung oder Ermittlung, die Anzahl von Erregungsimpulsen oder die Ladezeit des Ladeimpulses, oder beide, initial zu wählen.

Das Attribut initial steht im Rahmen der Offenbarung insbesondere für einen Zeitpunkt einer Inbetriebnahme des Detektors oder für einen Wechsel des Kanals, der Flüssigkeit oder eines Betriebsmodus des Detektors.

Ein Wechsel liegt beispielsweise vor, wenn der Betriebsmodus von Priming zu Therapie wechselt, oder umgekehrt, oder wenn die zu überwachende Flüssigkeit wechselt, beispielsweise von Primingflüssigkeit zu Blut, oder umgekehrt, oder wenn der Schlauch gewechselt wird.

Mit jedem Wechsel kann der Effekt auftreten, dass sich die Kopplung und/ oder die Dämpfung des Ultraschalls verändert. Die oben genannte Korrelation bildet diese Veränderung empirisch ab, sodass die nach dem Wechsel initial gewählte Anzahl von Erregungsimpulsen und/ oder die Ladezeit des Ladeimpulses die Veränderung initial bereits kompensiert oder zumindest teilweise kompensiert.

Gemäß einer Weiterbildung ist der oben genannte, wenigstens eine Parameter eine Herstellerbezeichnung, Typenbezeichnung, ID, elektrische oder geometrische Nenngröße oder ein Material des Ultraschall-Senders, des Ultraschall-Empfängers oder des Kanals, oder es ist ein Volumenstrom oder eine Art der Flüssigkeit, beispielsweise "Priming-Flüssigkeit" oder "medizinische Lösung" oder "Blut", oder ein Betriebsmodus des Detektors, beispielsweise "in Behandlung" oder "in Vorbereitung einer Behandlung".

Bei einer Anpassung im Sinne einer Erhöhung der Ladezeit des Ladeimpulses nähert sich der Strom der Induktivität dem Sättigungsstrom an, woraus ein umso stärkeres Magnetfeld resultiert und in Folge die selbstinduzierte Spannung der Induktivität höher ist. Wird die Induktivität über die nachfolgenden Erregungsimpulse entladen, ist die Sendeenergie entsprechend der höheren selbstinduzierten Spannung der Induktivität erhöht.

In die gleiche Richtung - in Richtung der Erhöhung der Sendeenergie - ist eine Erhöhung der Anzahl der Erregungsimpulse wirksam.

Die jeweils gegenteilige Absenkung im Sinne einer Absenkung der Ladezeit des Ladeimpulses, bzw. der Anzahl der Erregungsimpulse, führt zu einer Absenkung der Sendeenergie.

Die Ladezeit des Ladeimpulses beträgt vorzugsweise etwa 15µs ± 5µs. Die Frequenz der Erregungsimpulse - zur Anregung des Ultraschall-Senders auf Resonanzfrequenz - beträgt vorzugsweise etwa 1 bis 5MHz, bevorzugt etwa 2MHz. Eine Pausendauer im Anschluss an die Abfolge von Impulsen und bis zum nächsten Ladeimpuls beträgt vorzugsweise etwa 300µs bis 500 µs, bevorzugt etwa 480µs. Die Zeitspanne des Ansteuersignals, bzw. Burstintervalls - das heißt, die Zeitspanne der Abfolge von Impulsen inklusive der darauffolgenden Pause - beträgt vorzugsweise etwa 50 bis 900µs, bevorzugt etwa 500µs. Eine Frequenz des Burstintervalls beträgt vorzugsweise 1 bis 15kHz, bevorzugt etwa 2kHz.

Vorzugsweise sind der Ultraschall-Sender und der Ultraschall-Empfänger auf einer Achse angeordnet, die durch eine Mitte des Behältnisses verläuft. So ist die Ultraschallstrecke kurz und die Dämpfung ist gering, sodass ein vergleichsweise starkes Empfangssignal bereits mit vergleichsweise geringer Sendeenergie möglich ist.

Vorzugsweise bilden der Ultraschall-Sender und der Ultraschall-Empfänger zwischen sich eine Aufnahme aus, in die der Kanal/ der Schlauch eingelegt und lagefixiert werden kann.

Vorzugsweise ist ein Spitzenwertgleichrichter vorgesehen, in dem das Empfangssignal verarbeitet wird.

Vorzugsweise ist ein Komparator vorgesehen, in den das Ausgangssignal des Spitzenwertgleichrichters und ein Referenzwert eingehen, wobei ein Komparator-Ausgangssignal eine Impulsbreite eines Rechtecksignals ist, das vom Empfangssignal und Referenzwert gebildet ist.

Vorzugsweise ist ein Mittelwert-Bildner vorgesehen, von dem zuvor ermittelte Impulsbreiten gemittelt werden.

Ein Ausgangssignal des Mittelwert-Bildners ist der oben genannte Mittelwert der Sequenz von Empfangssignalen.

Gemäß einer Weiterbildung ist die Steuereinheit ausgestaltet, periodisch das Empfangssignal, vorzugsweise das abgeleitete Signal, besonders bevorzugt den Mittelwert, zu bilden, mit dem Zielwert oder dem Zielwertintervall zu vergleichen und die Anzahl von Erregungsimpulsen oder die Ladezeit des Ladeimpulses, oder beide, anzupassen, insbesondere wenn das Empfangssignal, vorzugsweise das abgeleitete Signal, besonders bevorzugt der Mittelwert, das Zielwertintervall verlässt. So wird die Sendeenergie nur angepasst, wenn der Mittelwert des Empfangssignals das Zielwert-Intervall verlässt. Das Empfangssignal/ abgeleitete Signal/ der Mittelwert kann somit innerhalb des Zielwert-Intervalls driften, ohne dass die Sendeenergie angepasst wird, was den Aufwand verringert.

Gemäß einer Weiterbildung ist die Steuereinheit ausgestaltet, die Anzahl von Erregungsimpulsen oder die Ladezeit des Ladeimpulses, oder beide, insbesondere mit einer vorbestimmten Schrittweite, anzupassen, bis das Empfangssignal/ abgeleitete Signal/ der Mittelwert auf den Zielwert oder in das Zielwert-Intervall geführt ist.

Gemäß einer Weiterbildung ist die Steuereinheit ausgestaltet, die Anzahl von Erregungsimpulsen oder die Ladezeit des Ladeimpulses, oder beide, zu erhöhen, wenn das Empfangssignal/ abgeleitete Signal/ der Mittelwert unter dem Zielwert oder einer unteren Grenze des Zielwertintervalls liegt, und die Anzahl von Erregungsimpulsen oder die Ladezeit des Ladeimpulses, oder beide, abzusenken, wenn das Empfangssignal/ abgeleitete Signal/ der Mittelwert über dem Zielwert oder einer oberen Grenze des Zielwertintervalls liegt.

Gemäß einer Weiterbildung ist die Steuereinheit ausgestaltet, die Anzahl von Erregungsimpulsen oder die Ladezeit des Ladeimpulses, oder beide, mit einer jeweils vorbestimmten, oberen Grenze zu vergleichen und nur zu erhöhen, wenn sie unterhalb ihrer jeweils vorbestimmten, oberen Grenze liegt oder liegen. So ist sichergestellt, dass eine obere Grenze der Sendeenergie nicht überschritten wird und die Flüssigkeit nicht geschädigt wird.

Alternativ oder ergänzend ist die Steuereinheit ausgestaltet, die Anzahl von Erregungsimpulsen oder die Ladezeit des Ladeimpulses, oder beide, mit einer jeweils vorbestimmten, unteren Grenze zu vergleichen und nur abzusenken, wenn sie oberhalb ihrer jeweils vorbestimmten, unteren Grenze liegt oder liegen. So ist sichergestellt, dass eine untere Grenze der Sendeenergie nicht unterschritten wird, um zu gewährleisten, dass ein ausreichend verfügbares Empfangssignal bereitgestellt wird.

Gemäß einer möglichen Weiterbildung ist die Steuereinheit ausgestaltet, eine Abweichung des Empfangssignals/ abgeleiteten Signals/ Mittelwertes vom Zielwert zu bilden, wobei die Steuereinheit eine Regeleinrichtung umfasst und eine Regelgröße der Regeleinrichtung der Mittelwert, eine Führungsgröße der Regeleinrichtung der Zielwert und Stellgrößen der Regeleinrichtung wahlweise die Anzahl von Erregungsimpulsen oder die Ladezeit des Ladeimpulses, oder beide, sind.

Gemäß der Offenbarung hat ein medizinisches Behandlungsgerät, insbesondere ein Dialysegerät, einen Detektor, der gemäß wenigstens einem Aspekt der vorangegangenen Beschreibung ausgebildet ist, wobei ein von der Flüssigkeit durchströmbarer oder durchströmter Kanal, insbesondere Schlauch, zwischen dem Ultraschall-Sender und dem Ultraschall-Empfänger angeordnet ist.

Ein offenbarungsgemäßes (computerimplementiertes) Verfahren zur Erkennung von Einschlüssen, insbesondere von Blasen, in einer extrakorporal strömenden Flüssigkeit, insbesondere in Blut oder medizinischer Lösung, ist für einen auf Ultraschallbasis arbeitenden Detektor vorgesehen. Der Detektor ist insbesondere gemäß wenigstens einem Aspekt der vorangegangenen Beschreibung ausgebildet und an einem medizinischen Behandlungsgerät vorgesehen, insbesondere an einem Dialysegerät. Das offenbarungsgemäße Verfahren hat folgende Schritte:
- Ansteuern eines Ultraschall-Senders des Detektors mit einem Ansteuersignal, das eine Abfolge von Impulsen umfasst, wobei die Abfolge einen Ladeimpuls mit einer Ladezeit und auf den Ladeimpuls folgend eine Anzahl von Erregungsimpulsen umfasst, mittels einer Steuereinheit,
- Senden eines Ultraschalls mit einer dem Ansteuersignal gemäßen Sendeenergie durch einen die Flüssigkeit führenden Kanal, insbesondere einen Schlauch, mittels dem Ultraschall-Sender,
- Empfangen des Ultraschalls und Bereitstellen eines Empfangssignals gemäß einer Empfangsenergie des Ultraschalls, mittels einem Ultraschall-Empfänger des Detektors,
- vorzugsweise Bilden eines vom Empfangssignal abgeleiteten Signals, besonders bevorzugt Bilden eines Mittelwertes des Empfangssignals als abgeleitetes Signal, und
- Anpassen der Sendeenergie in Abhängigkeit des Empfangssignals, um das Empfangssignal, bevorzugt das davon abgeleitete Signal, besonders bevorzugt den Mittelwert, auf einen Zielwert oder in ein Zielwert-Intervall zu führen, mittels der Steuereinheit. Offenbarungsgemäß erfolgt das Anpassen der Sendeenergie, indem die Anzahl von Erregungsimpulsen und/ oder die Ladezeit des Ladeimpulses des Ansteuersignals angepasst wird.

Gemäß einer Weiterbildung hat das Verfahren Schritte:
- Einlesen wenigstens eines Parameters des Detektors, des Kanals oder der Flüssigkeit, mittels der Steuereinheit,
- Aufrufen einer Korrelation, in der eine initiale Anzahl von Erregungsimpulsen und/ oder eine initiale Ladezeit des Ladeimpulses, insbesondere eine initiale Sendeenergie, in Abhängigkeit des wenigstens einen Parameters abgelegt ist oder sind, mittels der Steuereinheit,
- Wählen der initialen Anzahl von Erregungsimpulsen und/ oder initialen Ladezeit des Ladeimpulses, und
- Ansteuern des Ultraschall-Senders mit dem die initiale Anzahl von Erregungsimpulsen und/ oder initiale Ladezeit des Ladeimpulses aufweisenden Ansteuersignal, um das Empfangssignal, bevorzugt das davon abgeleitete Signal, besonders bevorzugt den Mittelwert, initial möglichst nahe an den Zielwert oder das Zielwert-Intervall zu führen, mittels der Steuereinheit.

Gemäß einer Weiterbildung des Verfahrens wird periodisch das Empfangssignal/ das abgeleitete Signal/ der Mittelwert mit dem Zielwert oder dem Zielwertintervall verglichen und die Anzahl von Erregungsimpulsen oder die Ladezeit des Ladeimpulses, oder beide, werden angepasst.

Gemäß einer Weiterbildung des Verfahrens erfolgt das Anpassen der Anzahl von Erregungsimpulsen oder der Ladezeit des Ladeimpulses, oder beider, insbesondere mit einer vorbestimmten Schrittweite, solange, bis das Empfangssignal/ das abgeleitete Signal/ der Mittelwert auf den Zielwert oder in das Zielwertintervall geführt ist.

Gemäß einer Weiterbildung des Verfahrens erfolgt ein Vergleichen des Empfangssignals/ des abgeleiteten Signals/ des Mittelwertes mit dem Zielwertintervall periodisch, und das Anpassen der Anzahl von Erregungsimpulsen oder der Ladezeit des Ladeimpulses, oder beider, erfolgt, sobald der Vergleich ergibt, dass das Empfangssignal/ das abgeleitete Signal/ der Mittelwert das Zielwertintervall verlässt.

Gemäß einer Weiterbildung des Verfahrens erfolgt ein Erhöhen der Anzahl von Erregungsimpulsen oder der Ladezeit des Ladeimpulses, oder beider, wenn das Empfangssignal/ das abgeleitete Signal/ der Mittelwert unter dem Zielwert oder einer unteren Grenze des Zielwertintervalls liegt, und ein Absenken der Anzahl von Erregungsimpulsen oder der Ladezeit des Ladeimpulses, oder beider, wenn das Empfangssignal/ das abgeleitete Signal/ der Mittelwert über dem Zielwert oder einer oberen Grenze des Zielwertintervalls liegt.

Gemäß einer Weiterbildung hat das Verfahren Schritte:
- Vergleichen der Anzahl von Erregungsimpulsen oder der Ladezeit des Ladeimpulses, oder beider, mit einer jeweils vorbestimmten, oberen Grenze, und
- Erhöhen der Anzahl von Erregungsimpulsen oder der Ladezeit des Ladeimpulses, oder beider, nur, wenn sie unterhalb ihrer jeweils vorbestimmten, oberen Grenze liegt oder liegen, mittels der Steuereinheit, und
- Vergleichen der Anzahl von Erregungsimpulsen oder der Ladezeit des Ladeimpulses, oder beider, mit einer jeweils vorbestimmten, unteren Grenze, und
- Absenken der Anzahl von Erregungsimpulsen oder der Ladezeit des Ladeimpulses, oder beider, nur, wenn sie oberhalb ihrer jeweils vorbestimmten, unteren Grenze liegt oder liegen, mittels der Steuereinheit.

Gemäß einer Weiterbildung hat das Verfahren wenigstens einen der folgenden Schritte:
- Abfragen, ob die Anpassung der Sendeenergie, anstatt auf Basis der Anpassung der Anzahl von Erregungsimpulsen, auf Basis der Anpassung der Ladezeit des Ladeimpulses fortgeführt werden soll, wenn das Vergleichen der Anzahl von Erregungsimpulsen mit ihrer vorbestimmten, oberen Grenze ergibt, dass die Anzahl von Erregungsimpulsen oberhalb ihrer vorbestimmten, oberen Grenze liegt, oder wenn das Vergleichen der Anzahl von Erregungsimpulsen mit ihrer vorbestimmten, unteren Grenze ergibt, dass die Anzahl von Erregungsimpulsen unterhalb ihrer vorbestimmten, unteren Grenze liegt;
- Abfragen, ob die Anpassung der Sendeenergie, anstatt auf Basis der Anpassung der Ladezeit des Ladeimpulses, auf Basis der Anpassung der Anzahl von Erregungsimpulsen fortgeführt werden soll, wenn das Vergleichen der Ladezeit des Ladeimpulses mit ihrer vorbestimmten, oberen Grenze ergibt, dass die Ladezeit des Ladeimpulses oberhalb ihrer vorbestimmten, oberen Grenze liegt, oder wenn das Vergleichen der Ladezeit des Ladeimpulses mit ihrer vorbestimmten, unteren Grenze ergibt, dass die Ladezeit des Ladeimpulses unterhalb ihrer vorbestimmten, unteren Grenze liegt.

Ein computerlesbares Speichermedium umfasst gemäß der Offenbarung Befehle, die bei einer Ausführung durch einen Computer diesen veranlassen, die Verfahrensschritte des Verfahrens für den Detektor gemäß wenigstens einem der vorbeschriebenen Aspekte auszuführen.

Im Folgenden werden bevorzugte Ausführungsformen der Erfindung anhand von Zeichnungen näher erläutert. Es zeigen:
Figur 1 in einer schematischen Darstellung ein offenbarungsgemäßes medizinisches Behandlungsgerät mit einem offenbarungsgemäßen Detektor mit einem Ultraschall-Sender und einem Ultraschall-Empfänger, sowie mit einer Steuereinheit,
Figur 2 in einer schematischen Darstellung ein Ansteuersignal für den Ultraschall-Sender gemäß Figur 1,
Figur 3 in einer schematischen Darstellung das Ansteuersignal gemäß Figur 2 mit einem Ladeimpuls mit angepasster Ladezeit,
Figur 4 in einer schematischen Darstellung das Ansteuersignal gemäß Figur 2 mit einer angepassten Anzahl von Erregungsimpulsen,
Figur 5 in einer schematischen Darstellung das Ansteuersignal gemäß Figur 2 und ein daraus resultierendes Empfangssignal des Ultraschall-Empfängers,
Figur 6 in einer schematischen Darstellung das Ansteuersignal gemäß Figur 5 mit einer höheren Anzahl von Erregungsimpulsen und das daraus resultierende Empfangssignal des Ultraschall-Empfängers,
Figur 7 in einer schematischen Darstellung ein Blockdiagramm des Detektors gemäß Figur 1 mit einer Rückführung und Bewertung eines vom Empfangssignal abgeleiteten Signals und einer Anpassung der Ladedauer oder Ladezeit eines Ladeimpulses und/ oder der Anzahl der Erregungsimpulse des Ansteuersignals,
Figur 8 in einer schematischen Darstellung drei unterschiedliche Verläufe des Empfangssignals, die sich bei gegebenem Ansteuersignal und gegebenem Flüssigkeitsstrom für unterschiedlich starke Dämpfungen/ Kopplungen der Ultraschallstrecke ergeben,
Figur 9 in einer schematischen Darstellung ein Ablaufdiagramm eines offenbarungsgemäßen Verfahrens mit einer Anpassung der Anzahl Erregungsimpulse des Ansteuersignals für den Detektor, und
Figur 10 in einer schematischen Darstellung ein Ablaufdiagramm eines offenbarungsgemäßen Verfahrens mit einer Anpassung der Ladezeit des Ladeimpulses des Ansteuersignals für den Detektor.

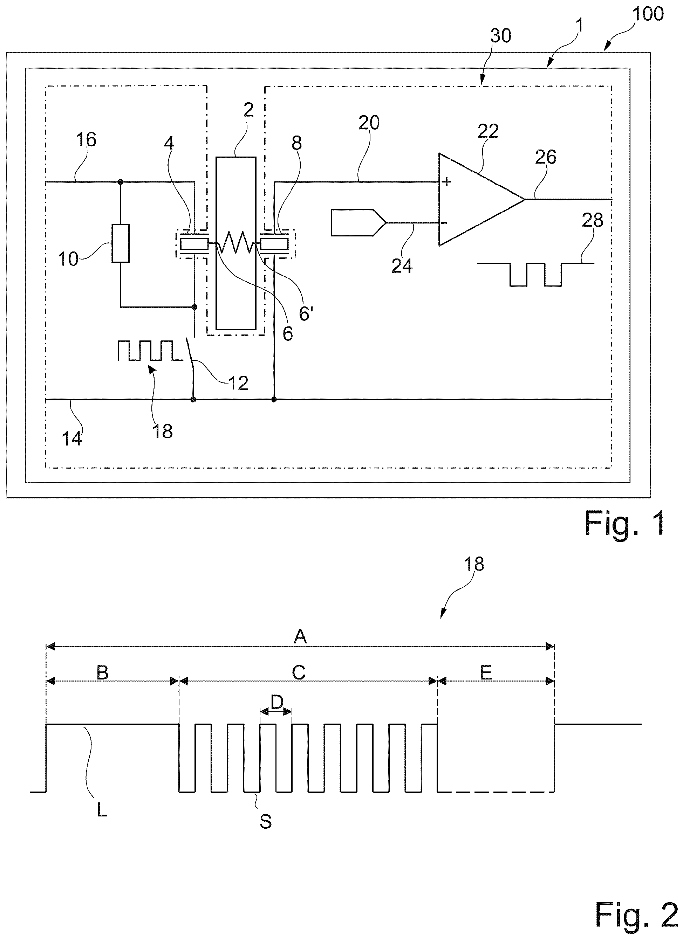

Gemäß Figur 1 ist ein Detektor 1 zur Erkennung von Luft in einer Flüssigkeit, in Form von Blut eines Dialysepatienten, vorgesehen. Der Detektor 1 ist eine Komponente eines als Dialysemaschine ausgebildeten, medizinischen Behandlungsgerätes 100, das lediglich grob schematisch dargestellt ist. Blut durchströmt einen Schlauch 2 und der Detektor 1 weist - jeweils in Form eines Piezoelements - einen Ultraschall-Sender 4 zum Senden eines Ultraschalls 6 durch den Schlauch 2 und einen Ultraschall-Empfänger 8 zum Empfangen von Ultraschall auf. Der Ultraschall-Sender 4, der Schlauch 2 mit der Flüssigkeit und der Ultraschall-Empfänger 8 bilden eine Ultraschallstrecke. Zum Aussenden des Ultraschalls wird der Ultraschall-Sender 4 mit einer elektrischen Spannung über eine elektrisch parallel zum Ultraschall-Sender 4 angeordnete Induktivität 10 beaufschlagt.

Der Ultraschall-Sender 4 und die Induktivität 10 sind elektrisch parallel über einen Schalter 12 mit einer Masse 14 verbindbar. Des Weiteren sind sie elektrisch parallel an eine Spannungsquelle 16 angeschlossen. Eine Selbstinduktionsspannung der Induktivität 10 entsteht beim Öffnen des Schalters 12 und liegt dann am Ultraschall-Sender 4 an. Hochfrequentes Öffnen und Schließen des Schalters 12 führt so zur hochfrequenten Spannungsänderung am Ultraschall-Sender 4 und zu einem entsprechenden Ultraschall 6. Diese Ansteuerung und damit Anregung des Ultraschall-Senders 4 erfolgt gemäß einem Ansteuersignal 18 einer Steuereinheit 30 des Detektors 1.

Der ausgesendete Ultraschall 6, der den Schlauch 2 und die darin strömende Flüssigkeit (Blut) durchlaufen hat, wird vom Ultraschall-Empfänger 8 empfangen und in ein elektrisches Empfangssignal 20 gewandelt. Das Empfangssignal 20 wird von einer Vergleichseinheit 22 (Komparator) mit einem Referenzwert/ einer Referenzspannung 24 verglichen und bewertet. An einem Ausgang 26 der Vergleichseinheit 22 steht somit ein vom Empfangssignal 20 abgeleitetes (grob schematisch dargestelltes) Ausgabesignal 28 zur Verfügung, welches zumindest die qualitative Auskunft gibt, ob sich in der Ultraschallstrecke "Luft" oder "keine Luft" (also nur Flüssigkeit) befindet. Auch quantitative Aussagen über das Luftvolumen lassen sich herleiten.

In Figur 2 ist das Ansteuersignal 18 über die Zeit dargestellt. Das Ansteuersignal 18 hat eine zeitliche Struktur, das heißt, die Spannung des Ansteuersignals 18 variiert zeitabhängig, sodass es eine Abfolge von Impulsen L, S aufweist.

Die Abfolge von Impulsen umfasst einen Ladeimpuls L mit einer Ladezeit B und eine auf den Ladeimpuls L folgende Anzahl C von Erregungsimpulsen S.

Das Ansteuersignal 18 ist in Burst-Intervalle A strukturiert. Eines der Burst-Intervalle A dauert beispielsweise zwischen 50 und 900 µs. Eine Wiederholfrequenz der Burst-Intervalle A beträgt beispielsweise 1 bis 15 kHz.

Das Burst-Intervall A selbst umfasst gemäß Figur 2 drei aufeinanderfolgende Abschnitte mit unterschiedlichem Spannungsverlauf: Zuerst den Ladeimpuls L mit der Ladezeit B, in der der Schalter 12 geschlossen ist, sodass an der Induktivität 10 die Spannung der Spannungsquelle 16 aus Figur 1 anliegt und die Induktivität 10 geladen wird. Die Ladezeit B der Induktivität 10 beträgt beispielsweise 5 bis 20 µs. Nach Ablauf der Ladezeit B folgt die Anzahl C von Erregungsimpulsen S mit einer Anregungsfrequenz D des Ultraschall-Senders 4 von beispielsweise 1 bis 5MHz. Hierbei sind gemäß Figur 2 beispielsweise acht Erregungsimpulsen S (Anzahl C=8) vorgesehen. Ein Abstand zwischen zwei Erregungsimpulsen S beträgt beispielsweise im Fall einer Anregungsfrequenz von 2MHz 500ns. Nach Ablauf der Erregungsimpulse S folgt eine Pause E ohne Erregungsimpulse und ohne Laden der Induktivität 10, bis die Ladezeit B eines neuen Burstintervalls A startet. Die Pause E beträgt beispielsweise 300 bis 500µs (beispielsweise 481µs). Das Burst-Intervall A ist somit der Abstand zweier Startzeitpunkte von aufeinander folgenden Ladeimpulsen L, bzw. Ladezeiten B.

Bezogen auf ein Burstintervall A steigt eine Stärke oder eine Energie des damit erzeugten Ultraschalls 6 sowohl mit der Ladezeit B des Ladeimpulses L für die Induktivität 10 als auch mit der Anzahl C der Erregungsimpulse S an. Umgekehrt sinkt die Stärke oder Energie des Ultraschalls 6 mit sinkender Ladezeit B des Ladeimpulses L, bzw. mit sinkender Anzahl C von Erregungsimpulsen S.

Eine Änderung der Ladezeit B des Ladeimpulses L oder der Anzahl C von Erregungsimpulsen S, oder beider, kann somit gemäß der Offenbarung genutzt werden, die Sendeenergie des vom Ultraschall-Sender 4 gesendeten Ultraschalls 6 zu ändern oder anzupassen, um Toleranzen in der Kopplung und/ oder Dämpfung der Ultraschallstrecke zu kompensieren.

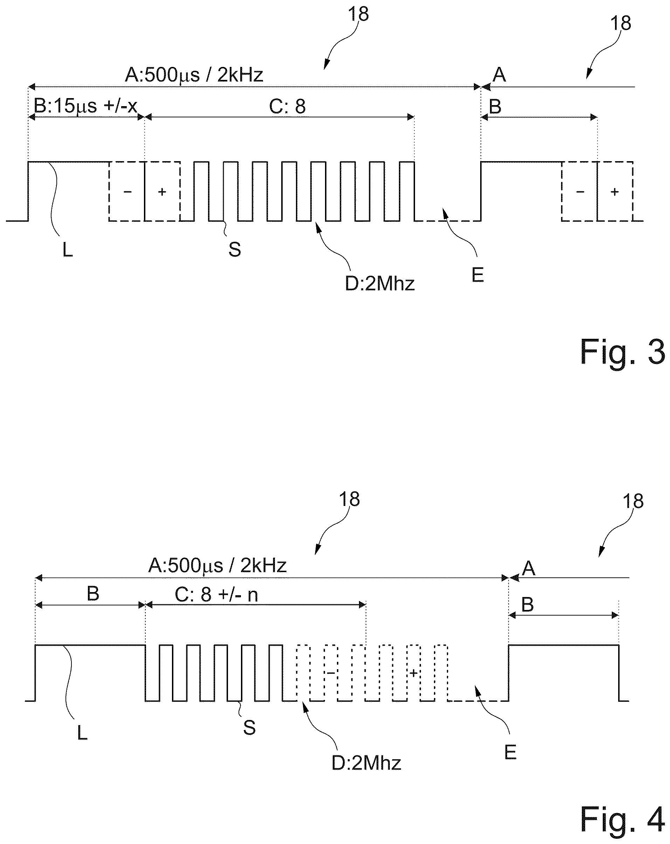

Figur 3 zeigt hierzu beispielhaft eine mögliche Änderung des Ansteuersignals 18 - und damit der Sendeenergie des Ultraschalls 6 - durch eine Änderung der Ladezeit B des Ladeimpulses L von 15 µs um ± x µs, bei ansonsten unveränderter Pause E, Frequenz D und Anzahl C der Erregungsimpulse S.

Figur 4 zeigt die Möglichkeit einer Erhöhung und einer Verringerung der Anzahl C von Erregungsimpulsen S um beispielsweise jeweils drei Erregungspulse S, ausgehend von acht Erregungspulsen S - und damit eine entsprechende Änderung/ Anpassung der aus dem Ansteuersignal 18 resultierenden Sendeenergie des Ultraschalls 6 - bei ansonsten unveränderter Frequenz D, Ladezeit B des Ladeimpulses L und Dauer des Burstintervalls A, bzw. Ansteuersignals 18.

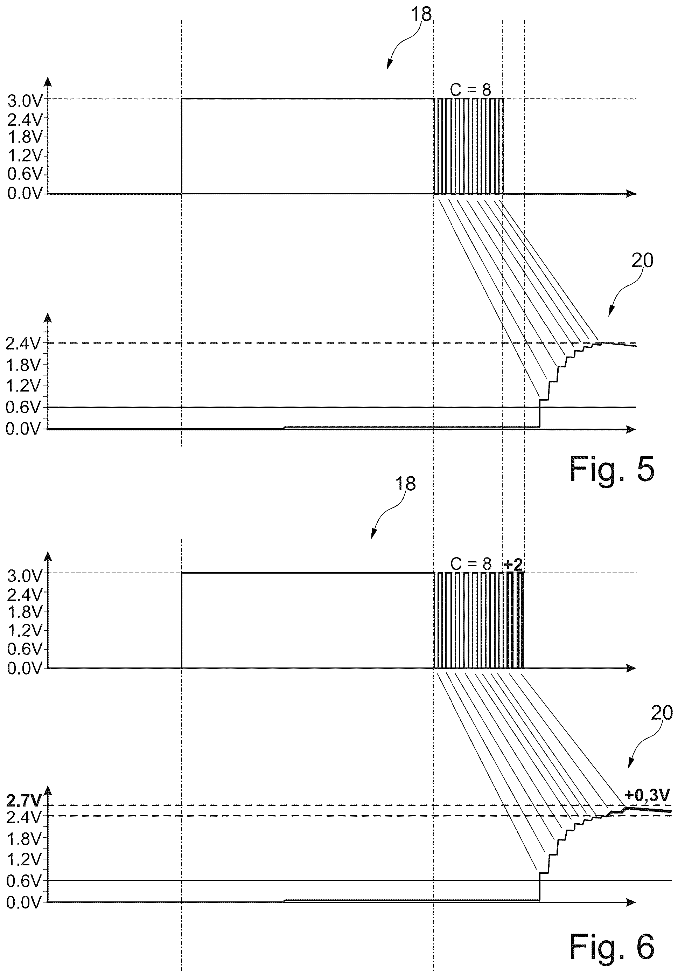

Figur 5 oben zeigt das Ansteuersignal 18 mit acht (C = 8) Erregungsimpulsen S in Abhängigkeit der Zeit. Figur 5 unten zeigt das auf dem empfangenen Ultraschall 6' basierende, vom Ultraschall-Empfänger 8 bereitgestellte Empfangssignal 20 in Abhängigkeit der Zeit. Verdeutlicht wird, dass jeder Erregungsimpuls S des oben dargestellten Burstsignals A die Sendeenergie des Ultraschalls 6 und somit auch eine Empfangsenergie des in Figur 5 unten dargestellten Empfangssignals 20 weiter erhöht. Die gemäß Figur 5 mit der Ladezeit B des Ladeimpulses L und der Anzahl C von acht Erregungsimpulsen S erreichte Stärke des Empfangssignals 20 beträgt 2.4 V.

Figur 6 zeigt ein angepasstes Ansteuersignal 18, basierend auf Figur 5. Die Anpassung besteht darin, dass die Anzahl C der Erregungsimpulse S von acht auf zehn erhöht wurde (+2). Das führt, wie bereits dargelegt, zu einem stärkeren Ultraschall 6. Gemäß Figur 6 unten erhöht sich damit auch die erreichte Stärke des Empfangssignals 20 von 2.4 V (vgl. Fig. 5) auf 2.7 V, also um +0.3 V.

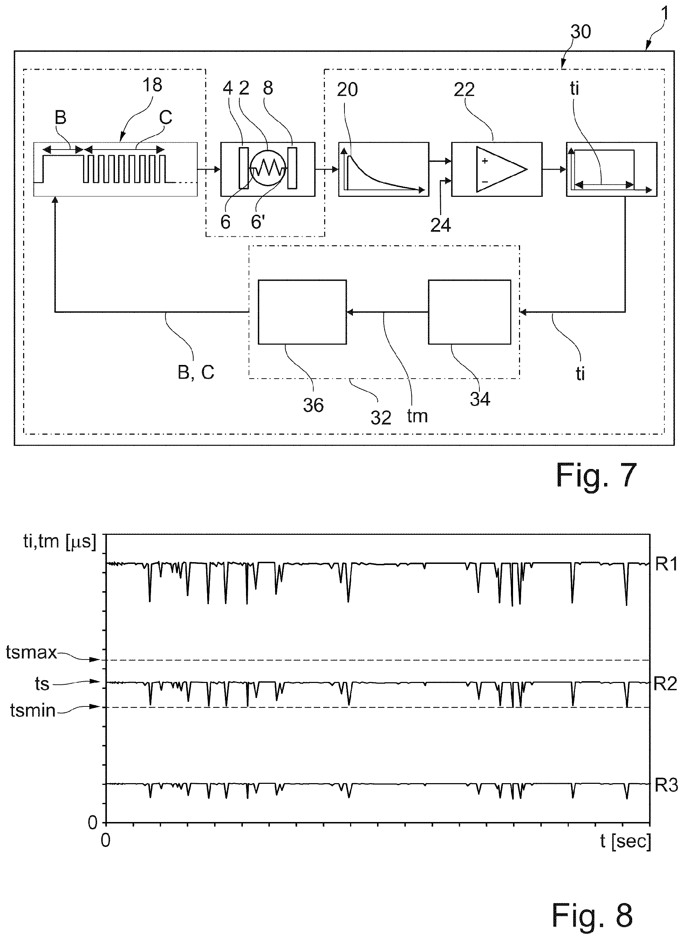

Figur 7 zeigt - unter Bezug auf Figur 1 und 2 - in einer schematischen Darstellung ein Blockdiagramm des Detektors 1. Ausgangspunkt ist das Ansteuersignal 18 gemäß Figur 2 mit der vorbeschriebenen Abfolge von Impulsen und der Ladezeit B des Ladeimpulses L und der Anzahl C der Erregungsimpulse S. Während der Ladezeit B erfolgt das Aufladen der Induktivität 10 gemäß Figur 1. Im Anschluss an die Ladezeit B erfolgt die hochfrequente Entladung der Induktivität 10 gemäß Figur 1 mit der Anzahl C der Erregungsimpulse S. Dementsprechend wird der Ultraschall-Sender 4 auf seine Resonanzfrequenz angeregt und er sendet Ultraschall 6 mit der dem Ansteuersignal 18 entsprechenden Sendeenergie aus. Der Ultraschall durchquert den Schlauch 2 mit strömender Flüssigkeit und erfährt dabei in Abhängigkeit von einer Güte der Kopplung, von Materialeigenschaften, von der Strömung und ggf. anderen Parametern eine Dämpfung. Der gedämpfte Ultraschall 6' trifft mit gegenüber der Sendeenergie geringerer Empfangsenergie auf den Ultraschall-Empfänger 8. Dieser stellt das der Empfangsenergie entsprechende Empfangssignal 20 bereit, das in einen Spitzenwertgleichrichter eingeht. Daraufhin wird das Signal auf den Komparator 22 gegeben, der den Vergleich mit dem Referenzwert/ der Referenzspannung 24 durchführt. Es resultiert ein vom Empfangssignal 20 abgeleitetes Signal in Form eines Rechtecksignals, dessen positive Pulsbreite ti erfasst wird.

Die vorbeschriebene Ansteuerung mit dem Ansteuersignal 18 des Ultraschall-Senders 4 erfolgt im gezeigten Ausführungsbeispiel periodisch mit der Wiederholfrequenz 2kHz des Ansteuersignals 18, das heißt, alle 500µs (des Burstintervalls A), wobei jedes Mal die o.g. positive Pulsbreite ti des Empfangssignals 20 erfasst wird.

Eine Sequenz der wiederholt erfassten, positiven Pulsbreiten ti geht in einen Microcontroller 32 der Steuereinheit 30 ein.

Eine Auswerteeinheit 34 des Microcontrollers 32 bildet aus den positiven Pulsbreiten ti der Sequenz eine statistische Größe.

Im gezeigten Ausführungsbeispiel ist diese statistische Größe ein Durchschnitt oder Mittelwert tm. In anderen Worten ausgedrückt wird von der Auswerteeinheit 34 aus einer Sequenz von Empfangssignalen 20 als statistische Größe der Mittelwert tm abgeleitet/ gebildet.

Eine Anpassungseinrichtung 36 des Microcontrollers 32 überprüft, ob dieser Mittelwert tm in einem vorbestimmten oder gewünschten Zielwert-Intervall tsmin, tsmax liegt (vgl. R2, Fig. 8). Ist das nicht der Fall, wird die Ladezeit B des Ladeimpulses L oder die Anzahl C der Erregungsimpulse S des Ansteuersignals 18 angepasst oder es werden die Ladezeit B und die Anzahl C angepasst. Aus dieser Anpassung resultiert eine entsprechende Änderung/ Anpassung der in die Ultraschallstrecke 4, 2, 8 eingebrachten Sendeenergie, was wiederum zu einem geänderten/ angepassten Empfangssignal 20 und in Folge zu einem geänderten/ angepassten Mittelwert tm führt. Die offenbarungsgemäße Anpassung wird beendet, wenn die Überprüfung durch die Anpassungseinrichtung 36 ergibt, dass der Mittelwert tm in das Zielwert-Intervall tsmin, tsmax geführt wurde.

Zur Verdeutlichung des Einflusses von Toleranzen der Ultraschallstrecke 4, 2, 8 sind in Figur 8 beispielhaft drei Signalverläufe dargestellt, die sich bei gegebenem Ansteuersignal 18 und Flüssigkeitsstrom für unterschiedliche Toleranzen und somit unterschiedlich starke Dämpfungen der Ultraschallstrecke 4, 2, 8 ergeben. Dargestellt ist der zeitliche Signalverlauf der positiven Impulsbreite ti. Zu erkennen ist, dass bei einer Passage von Blasen die positive Impulsbreite ti jeweils absinkt. Je größer die Blase, umso stärker ist die Absenkung.

Eine geringe Dämpfung des Ultraschalls 6 entlang der Ultraschallstrecke resultiert in einem starken Signalverlauf der positiven Impulsbreite ti, beispielsweise im Bereich R1. Im Bereich R2 hingegen liegt eine mittlere Dämpfung vor und im Bereich R3 liegt die stärkste Dämpfung vor, was verglichen zum Bereich R1 zu entsprechend abgeschwächten Signalverläufen der positiven Impulsbreite ti und auch zu entsprechend abgeschwächten Absenkungen führt.

Gemäß Figur 8 ist der Bereich R2 derjenige, in dem der Mittelwert tm des Empfangssignals 20 innerhalb des Zielwert-Intervalls tsmin, tsmax liegt. Überschreitet oder trifft der Mittelwert tm die obere Grenze tsmax, erfolgt die oben beschriebene, offenbarungsgemäße Anpassung im Sinne einer Absenkung der Ladezeit B des Ladeimpulses L und/ oder einer Verringerung der Anzahl C von Erregungsimpulsen S. Im umgekehrten Fall, sollte der Mittelwert tm die untere Grenze tsmin treffen oder unterschreiten, erfolgt die oben beschriebene Anpassung im Sinne einer Erhöhung der Ladezeit B des Ladeimpulses L und/ oder einer Erhöhung der Anzahl C von Erregungsimpulsen S.

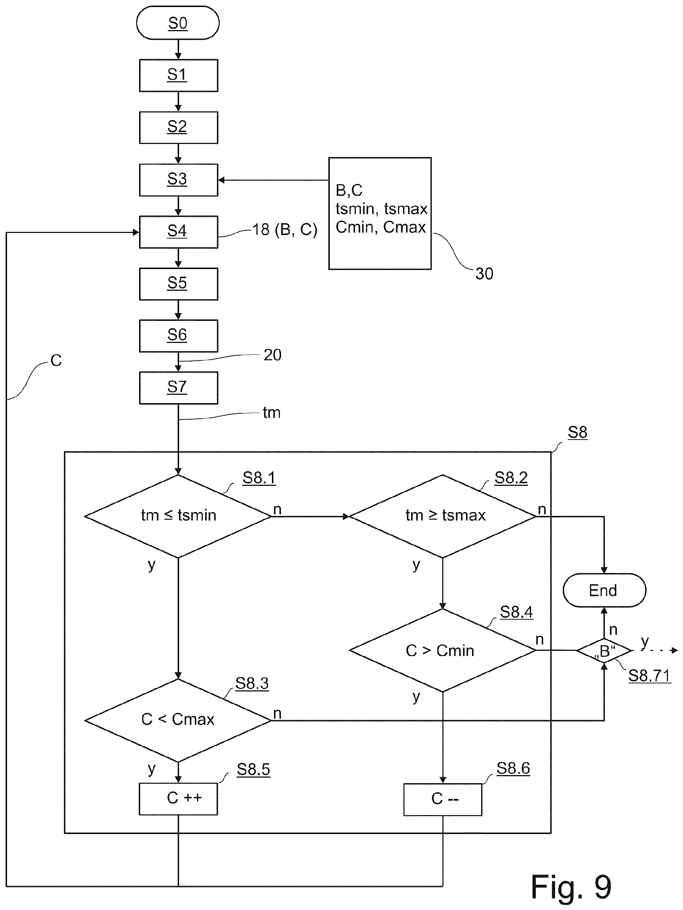

Figur 9 zeigt in einer schematischen Darstellung ein Ablaufdiagramm eines Verfahrens zur Erkennung von Einschlüssen in einer extrakorporal strömenden Flüssigkeit für den Detektor 1 auf Ultraschallbasis, bei dem diese Anpassung der Sendeenergie/ des Ansteuersignals 18 offenbarungsgemäß erfolgt. Die Beschreibung der Figur 9 erfolgt auch mit Bezug zur Figur 7.

Zunächst erfolgt der Schritt Start oder Initialisierung **S0** des Verfahrens, beispielsweise durch eine entsprechende manuelle Eingabe in eine Benutzerschnittstelle und/ oder durch Erfassung des zwischen Ultraschall-Sender 4 und Ultraschall-Empfänger 8 eingelegten Schlauchs 2.

Es folgt der Schritt Einlesen **S1** wenigstens eines Parameters, welcher den Detektor 1, den Kanal 2, die Flüssigkeit und/ oder einen Flüssigkeitsstrom charakterisiert, beispielsweise durch manuelle Eingabe in eine Benutzerschnittstelle oder durch Erfassung über eine Erfassungseinheit oder durch Ermittlung.

Es folgt der Schritt Aufrufen **S2** einer, insbesondere empirisch ermittelten, Korrelation 30. In der Korrelation ist/ sind in Abhängigkeit des wenigstens einen Parameters eine initiale Anzahl C Erregungsimpulse S und/ oder eine initiale Ladezeit B des Ladeimpulses L abgelegt.

Die Korrelation 30 umfasst im gezeigten Ausführungsbeispiel Parameter der Ultraschallstrecke, welche Einfluss auf die Dämpfung des Ultraschalls haben und die strömende Flüssigkeit charakterisieren. Beispiele hierfür sind: eine Art des Betriebsmodus des Detektors 1 oder der Behandlungsmaschine 100, wie beispielsweise Priming oder Blutbehandlung oder Infusion; die Art der zu überwachenden Flüssigkeit, wie beispielsweise Primingflüssigkeit oder Blut oder medizinische Lösung; der Volumenstrom der Flüssigkeit; der Typ, die Nenngröße, das Material des Schlauchs; und dergleichen.

Die Korrelation 30 umfasst zudem im gezeigten Ausführungsbeispiel in Abhängigkeit des wenigstens einen Parameters die untere und obere Grenze der Sendeenergie, aufgeschlüsselt auf die Ladezeit B des Ladeimpulses L und die Anzahl oder Impulsanzahl C der Erregungsimpulse S. Diese Grenzen sind Bmin, Bmax für die Grenzen der Ladezeit B des Ladeimpulses L und Cmin, Cmax für die Grenzen der Anzahl C von Erregungsimpulsen S.

Darüber hinaus umfasst die Korrelation 30 im gezeigten Ausführungsbeispiel die untere und obere Grenze tsmin, tsmax des Zielwert-Intervalls des abgeleiteten Signals/ des Mittelwertes tm, in Abhängigkeit des wenigstens einen Parameters, insbesondere in Abhängigkeit des Betriebsmodus, der Flüssigkeit und des Flüssigkeitsstroms.

Es folgt der Schritt Wählen **S3** der initialen Anzahl C der Erregungsimpulse S und/ oder der initialen Ladezeit B des Ladeimpulses L in Abhängigkeit des wenigstens einen Parameters.

Es folgt der Schritt Ansteuern **S4** des Ultraschall-Senders 4 mit dem die initiale Anzahl C und/ oder die initiale Ladezeit B aufweisenden Ansteuersignal 18, was zur Anregung des Ultraschall-Senders 4 auf seine Resonanzfrequenz führt.

Es folgt der Schritt Senden **S5** des Ultraschalls 6 mit der dem Ansteuersignal 18 gemäßen Sendeenergie von dem Ultraschall-Sender 4, durch den Schlauch 2 und die Flüssigkeit, zu dem Ultraschall-Empfänger 8.

Der Ultraschall-Empfänger 8 empfängt und wandelt den Ultraschall 6' in das Empfangssignal 20 und es folgt der Schritt Bereitstellen **S6** des Empfangssignals 20 gemäß der Empfangsenergie des empfangenen Ultraschalls 6'.

Es folgt die Verarbeitung des Empfangssignals 20 durch den Spitzenwertgleichrichter und der Vergleich dieses Signals mit dem Referenzwert 24 durch den Komparator 22.

Der Komparator 22 liefert, wie bereits erwähnt, die positive Impulsbreite ti zu dem jeweiligen Empfangssignal 20 (vgl. Fig. 7).

Nach Ablauf einer Sequenz von Ansteuersignalen 18, bzw. Burstintervallen A (vgl. Fig.2), folgt der Schritt Bilden **S7** des vom Empfangssignal 20 abgeleiteten Signals tm durch die Auswerteeinheit 34 gemäß Figur 7. Allgemein ausgedrückt erfolgt das durch eine statistische Auswertung der positiven Impulsbreiten ti der Sequenz von Empfangssignalen 20.

Die statistische Auswertung kann insbesondere das Bilden einer statistischen Größe, insbesondere eines Mittelwertes, eines Medians, eines Maximalwertes und/ oder einer Standardabweichung umfassen.

Im konkreten Ausführungsbeispiel gemäß Figur 9 wird der Mittelwert tm als statistische Größe/ als abgeleitetes Signal gebildet.

In Folge muss überprüft werden, ob der wie vorbeschrieben abgeleitete Mittelwert tm bereits in dem Zielwert-Intervall tsmin, tsmax liegt, und demzufolge keine Anpassung der Sendeenergie mehr erfolgen muss, oder ob er außerhalb des Zielwert-Intervalls tsmin, tsmax liegt, und demzufolge die Anzahl C der Erregungsimpulse S und/ oder die Ladezeit B des Ladeimpulses L angepasst werden muss.

Diese Überprüfung erfolgt innerhalb des Schrittes Anpassen **S8** der Sendeenergie, um den Mittelwert tm in das Zielwert-Intervall tsmin, tsmax zu führen.

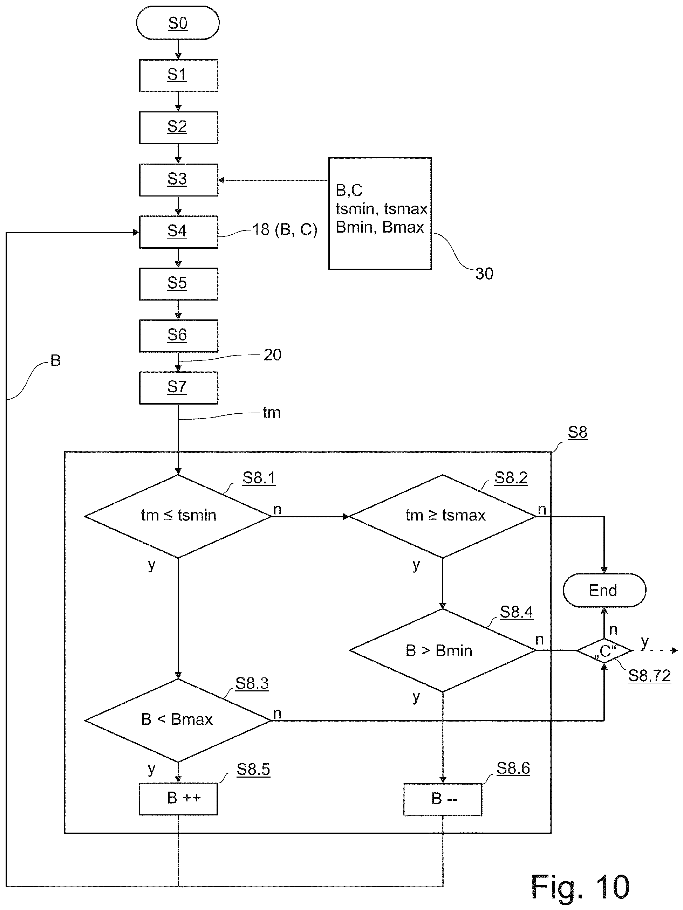

Der Schritt **S8** umfasst hierzu eine Schrittfolge **S8.1** bis **S8.6,** die zur Übersichtlichkeit im Folgenden allein mittels der Anzahl C der Erregungsimpulse S durchgeführt und beschrieben wird. Die Schrittfolge S8.1 bis S8.6 kann alternativ oder ergänzend mit der Ladezeit B des Ladeimpulses L und ihren oben genannten Grenzen Bmin, Bmax erfolgen, was Figur 10 verdeutlicht.

In dem in den Figuren 9 und 10 gezeigten Ausführungsbeispiel ist das Zielwert-Intervall tsmin, tsmax so definiert, dass dessen untere Grenze tsmin und obere Grenze tsmax nicht eingeschlossen sind.

Zunächst erfolgt der Schritt Abfrage **S8.1,** ob der Mittelwert tm kleiner-gleich (≤) der aus der Korrelation 30 geladenen unteren Grenze tsmin ist. Falls die untere Grenze tsmin eingeschlossen sein sollte, würde hier abgefragt, ob der Mittelwert tm kleiner (<) ist als die untere Grenze tsmin.

Ergibt die Abfrage **S8.1,** dass der Mittelwert tm größer (>) ist (n) als die untere Grenze tsmin, erfolgt der Schritt Abfrage **S8.2,** ob der Mittelwert tm größer-gleich (≥) der aus der Korrelation 30 geladenen oberen Grenze tsmax ist. Falls die obere Grenze tsmax eingeschlossen sein sollte, würde hier abgefragt, ob der Mittelwert tm größer (>) ist als die obere Grenze tsmax.

Ergibt diese Abfrage **S8.2,** dass der Mittelwert tm kleiner (<) ist als die obere Grenze tsmax (n), so liegt der Mittelwert tm folglich bereits innerhalb des Zielwert-Intervalls tsmin, tsmax. Die Sendeenergie liegt somit bereits mit den zuvor initial gewählten Werten der Anzahl C der Erregungsimpulse S und der Ladezeit B des Ladeimpulses L innerhalb ihrer Grenzen und der Schritt Anpassen **S8** der Sendeenergie kann mit dem Schritt **End** beendet werden.

Ergibt die o.g. Abfrage **S8.1** hingegen, dass der Mittelwert tm kleiner-gleich (≤) der unteren Grenze tsmin (y) ist, kann das geändert werden/ kann der Mittelwert tm in das Zielwert-Intervall tsmin, tsmax geführt werden, indem die Anzahl C der Erregungsimpulse S erhöht wird.

Zunächst muss aber überprüft werden, ob diese Erhöhung der Anzahl C der Erregungsimpulse S zulässig ist. Hierzu erfolgt die Abfrage **S8.3,** ob die Anzahl C der Erregungsimpulse S kleiner ist als ihre aus der Korrelation 30 geladene obere Grenze Cmax.

Ergibt diese Abfrage **S8.3,** dass die Anzahl C der Erregungsimpulse S größer-gleich (≥) der oberen Grenze Cmax (n) ist, bedeutet das, dass die Anzahl C der Erregungsimpulse S nicht weiter erhöht werden kann.

Es erfolgt das Ende der Anpassung der Sendeenergie auf Basis der Anzahl C der Erregungsimpulse S.

Da der Mittelwert tm aber noch unterhalb seiner unteren Grenze tsmin liegt (vgl. (y) des obigen Schrittes S8.1), kann die weiterhin notwendige Erhöhung der Sendeenergie des Ansteuersignals 18 im Folgenden optional oder zusätzlich mittels der Erhöhung der Ladezeit B des Ladeimpulses L erfolgen. Es erfolgt deshalb eine Abfrage **S8.7,** ob die Anpassung der Sendeenergie auf Basis der Anpassung der Ladezeit B des Ladeimpulses L fortgeführt werden soll.

Die Anpassung der Ladezeit B des Ladeimpulses L kann in vergleichsweise kleinen Schritten, quasi kontinuierlich, erfolgen. Demgegenüber kann die Anpassung der Anzahl C der Erregungsimpulse S in ganzzahligen und damit vergleichsweise großen Schritten erfolgen. Dieser Unterschied in den Schrittweiten kann vorteilhaft genutzt werden. Beispielsweise kann die Anpassung der Ladezeit B des Ladeimpulses L in einem Zeitraum bis zu einer nächsten Anpassung der Anzahl C der Erregungsimpulse S genutzt werden, um die Sendeenergie des Ansteuersignals 18 in kleinen Schritten, quasi kontinuierlich, zu erhöhen oder abzusenken. Ergänzend oder alternativ kann das Ansteuersignal 18 je nach Abstand des abgeleiteten Signals/ des Mittelwertes tm zum Zielwert-Intervall tsmin, tsmax mit großer Schrittweite durch Anpassung der Anzahl C der Erregungsimpulse S, oder mit feiner, quasi kontinuierlicher Schrittweite durch Anpassung der Ladezeit B des Ladeimpulses L angepasst werden. Ergänzend oder alternativ kann die Anpassung der Ladezeit B des Ladeimpulses L genutzt werden, um das abgeleitete Signal/ den Mittelwert tm innerhalb des Zielwert-Intervalls tsmin, tsmax oder nahe an dessen Grenzen tsmin, tsmax anzupassen, insbesondere, wenn das Zielwert-Intervall sehr eng ist.

Ergibt die o.g. Abfrage **S8.3** jedoch, dass die Anzahl C der Erregungsimpulse S kleiner (<) ist als ihre obere Grenze Cmax (y), so kann die Anzahl C der Erregungsimpulse S weiter erhöht werden, um so die Sendeenergie und damit den Mittelwert tm zu erhöhen.

Die Anzahl C der Erregungsimpulse S wird folglich im Schritt **S8.5** um einen festgelegten Schritt erhöht und die neue/ erhöhte Anzahl C der Erregungsimpulse S geht erneut ein in den Schritt Ansteuern **S4** des Ultraschall-Senders 4 mit dem die Anzahl C der Erregungsimpulse S aufweisenden Ansteuersignal 18.

Ergibt die oben genannte Abfrage **S8.2,** dass der Mittelwert tm größer-gleich (≥) der oberen Grenze tsmax (y) ist, so liegt der Mittelwert tm folglich noch nicht im Zielwert-Intervall tsmin, tsmax, sondern oberhalb davon.

Die dem Mittelwert tm zugrundeliegende Sendeenergie könnte somit durch eine Verringerung der Anzahl C der Erregungsimpulse S abgesenkt werden, um den Mittelwert tm ins Zielwert-Intervall tsmin, tsmax zu führen. Hierzu muss aber zunächst geprüft werden, ob die Anzahl C der Erregungsimpulse S oberhalb ihrer aus der Korrelation 30 herangezogenen unteren Grenze Cmin liegt, was im Abfrageschritt **S8.4** erfolgt.

Ergibt diese Abfrage **S8.4,** dass die Anzahl C der Erregungsimpulse S kleiner-gleich (≤) ihrer unteren Grenze Cmin (n) ist, bedeutet das, dass die Anzahl C der Erregungsimpulse S nicht weiter abgesenkt werden kann.

Es erfolgt das Ende der Anpassung der Sendeenergie auf Basis der Anzahl C der Erregungsimpulse S und eine Abfrage S8.7, ob die Anpassung der Sendeenergie auf Basis der Anpassung der Ladezeit B des Ladeimpulses L fortgeführt werden soll.

Da der Mittelwert tm aber noch oberhalb seiner oberen Grenze tsmax liegt (vgl. (y) des Schrittes S8.2), kann die weiterhin notwendige Absenkung der Sendeenergie im Folgenden optional mittels der Absenkung der Ladezeit B des Ladeimpulses L erfolgen.

Ergibt die Abfrage **S8.4** hingegen, dass die Anzahl C der Erregungsimpulse S größer (>) ist als ihre untere Grenze Cmin (y), so kann die Anzahl C der Erregungsimpulse S weiter abgesenkt werden, um so die Sendeenergie des Ansteuersignals 18 und damit den Mittelwert tm abzusenken.

Die Anzahl C der Erregungsimpulse S wird folglich im Schritt **S8.6** um einen festgelegten Schritt abgesenkt und die neue/ abgesenkte Anzahl C der Erregungsimpulse S geht erneut ein in den Schritt Ansteuern **S4** des Ultraschall-Senders 4 mit dem die Anzahl C aufweisenden Ansteuersignal 18.

Die Anpassung der Anzahl C der Erregungsimpulse S und/ oder Ladezeit B des Ladeimpulses L mittels der Schritte S8.1 bis S8.6 erfolgt so lange, bis der Mittelwert tm ins Zielwert-Intervall tsmin, tsmax geführt wurde.

Figur 10 zeigt alternativ oder ergänzend zum Verfahren gemäß Figur 9 die Anpassung der Sendeenergie des Ansteuersignals 18 durch Anpassung der Ladezeit B des Ladeimpulses L des Ansteuersignals 18. Die beiden Anpassungen mittels der Anzahl C der Erregungsimpulse S (Figur 9) und mittels der Ladedauer B des Ladeimpulses L (Figur 10) sind über die Abfrage S8.7, genauer gesagt über die Abfragen S8.71 und S8.72, verknüpft.

Offenbart ist ein Detektor zur Erkennung von Einschlüssen in einer strömenden Flüssigkeit, insbesondere in einem Schlauch mittels Ultraschall, bei dem durch eine variable Anpassung einer Sendeleistung oder Sendeenergie des Ultraschallsignals mittels Anpassung oder Regelung der Ladedauer einer Induktivität und/oder mittels Anpassung oder Regelung der Anzahl von Erregungsimpulsen Toleranzen und Dämpfungen des Ultraschallsignals ausgeglichen werden können.

### Bezugszeichenliste

- 1: Detektor
- 2: Schlauch
- 4: Ultraschall-Sender
- 6: Ultraschall gesendet
- 6': Ultraschall empfangen
- 8: Ultraschall-Empfänger
- 10: Induktivität
- 12: Schalter
- 14: Masse
- 16: Spannungsquelle
- 18: Ansteuersignal
- 20: Empfangssignal
- 22: Komparator
- 24: Referenzwert
- 26: Ausgang
- 28: Ausgabesignal
- 30: Steuereinheit
- 32: Mikrocontroller
- 34: Auswerteeinheit
- 36: Anpassungseinrichtung
- 100: medizinisches Behandlungsgerät

- A: Burst-Intervall
- L: Ladeimpuls
- B: Ladezeit Ladeimpuls
- S: Erregungsimpuls
- C: Anzahl Erregungsimpulse
- D: Ultraschall Anregungsfrequenz
- E: Pause
- ti: positive Pulsbreite
- tm: abgeleitetes Signal/ Mittelwert
- ts: Zielwert
- tsmin: Zielwert-Intervall untere Grenze
- tsmax: Zielwert-Intervall obere Grenze

- S0: Start
- S1: Einlesen Parameter
- S2: Aufrufen Korrelation
- S3: Wählen Anzahl/ Dauer
- S4: Ansteuern Ultraschall-Sender
- S5: Senden Ultraschall
- S6: Bereitstellen Empfangssignal
- S7: Bilden abgeleitetes Signal/ Mittelwert
- S8: Anpassen Anzahl und/ oder Ladezeit
- S8.1: Vergleichen untere Grenze Zielwert-Intervall
- S8.2: Vergleichen obere Grenze Zielwert-Intervall
- S8.3: Vergleichen obere Grenze Anzahl/ Ladezeit
- S8.4: Vergleichen untere Grenze Anzahl/ Ladezeit
- S8.5: Erhöhen Anzahl/ Ladezeit
- S8.6: Absenken Anzahl/ Ladezeit
- S8.71, S8.72: Abfrage Wechsel der Anpassung

## Patentansprüche

1. Detektor zur Erkennung von Einschlüssen in einer extrakorporal strömenden Flüssigkeit auf Basis von Ultraschall, insbesondere zur Erkennung von Blasen in Blut oder in medizinischer Lösung, mit einem Ultraschall-Sender (4), einem Ultraschall-Empfänger (8) und einer Steuereinheit (30), wobei:
- die Steuereinheit (30) ausgestaltet ist, den Ultraschall-Sender (4) mit einem Ansteuersignal (18) anzusteuern, das eine Abfolge von Impulsen (L, S) umfasst, wobei die Abfolge einen Ladeimpuls (L) mit einer Ladezeit (B) und eine auf den Ladeimpuls (L) folgende Anzahl (C) von Erregungsimpulsen (S) umfasst,
- der Ultraschall-Sender (4) ausgestaltet ist, einen Ultraschall (6) mit einer dem Ansteuersignal (18) gemäßen Sendeenergie durch einen die Flüssigkeit führenden Kanal (2), insbesondere Schlauch, zu senden,
- der Ultraschall-Empfänger (8) ausgestaltet ist, ein einer Empfangsenergie des Ultraschalls (6') gemäßes Empfangssignal (20) bereitzustellen, und
- die Steuereinheit (30) ausgestaltet ist, in Abhängigkeit des Empfangssignals (20) die Sendeenergie anzupassen, um das Empfangssignal (20), vorzugsweise ein davon abgeleitetes Signal (tm), auf einen Zielwert (ts) oder in ein Zielwert-Intervall (tsmin, tsmax) zu führen, **dadurch gekennzeichnet, dass** die Steuereinheit (30) ausgestaltet ist, die Anzahl (C) von Erregungsimpulsen (S) und/ oder die Ladezeit (B) des Ladeimpulses (L) des Ansteuersignals (18) anzupassen, um das Empfangssignal (20), vorzugsweise das davon abgeleitete Signal (tm), auf den Zielwert (ts) oder in das Zielwertintervall (tsmin, tsmax) zu führen.

2. Detektor nach Anspruch 1, **dadurch gekennzeichnet, dass** das abgeleitete Signal eine statistische Größe einer Sequenz von aufeinanderfolgenden Empfangssignalen (20) ist, insbesondere ein Mittelwert (tm), ein Median, ein Maximalwert und/ oder eine Standardabweichung.

3. Detektor nach Anspruch 1 oder 2, **dadurch gekennzeichnet, dass** die Steuereinheit (30) eine Korrelation, insbesondere eine Look-up-Tabelle und/ oder einen Algorithmus, der Anzahl (C) von Erregungsimpulsen (S) und/ oder der Ladezeit (B) des Ladeimpulses (L), insbesondere der resultierenden Sendeenergie, in Abhängigkeit wenigstens eines Parameters des Detektors (1) und/ oder des Kanals (2) und/ oder der Flüssigkeit umfasst, und die Steuereinheit (30) ausgestaltet ist, in Abhängigkeit des wenigstens einen Parameters die Anzahl (C) von Erregungsimpulsen (S) und/ oder die Ladezeit (B) des Ladeimpulses (L) initial zu wählen, um das Empfangssignal (20), vorzugsweise das abgeleitete Signal (tm), initial möglichst nahe an den Zielwert (ts) oder das Zielwert-Intervall (tsmin, tsmax) zu führen.

4. Detektor nach einem der vorhergehenden Ansprüche, **dadurch gekennzeichnet, dass** der Ultraschall-Sender (4) und der Ultraschall-Empfänger (8) jeweils von einem Piezoelement gebildet ist, wobei elektrisch parallel zum Ultraschall-Sender (4) eine Induktivität (10) vorgesehen ist, wobei die Abfolge von Impulsen (L, S) des Ansteuersignals (18) den Ladeimpuls (L) mit der Ladezeit (B) zum Laden der Induktivität (10) und die darauf folgende Anzahl (C) von Erregungsimpulsen (S) zum Erregen des Ultraschall-Senders (4) umfasst.

5. Detektor nach einem der vorhergehenden Ansprüche, **dadurch gekennzeichnet, dass** die Steuereinheit (30) ausgestaltet ist, periodisch das Empfangssignal (20), vorzugsweise das abgeleitete Signal (tm), mit dem Zielwert (ts) oder dem Zielwertintervall (tsmin, tsmax) zu vergleichen und die Anzahl (C) von Erregungsimpulsen (S) und/ oder die Ladezeit (B) des Ladeimpulses (L) anzupassen, insbesondere wenn das Empfangssignal (20), vorzugsweise das abgeleitete Signal (tm), das Zielwertintervall (tsmin, tsmax) verlässt.

6. Detektor nach einem der vorhergehenden Ansprüche, **dadurch gekennzeichnet, dass** die Steuereinheit (30) ausgestaltet ist, die Anzahl (C) von Erregungsimpulsen (S) oder die Ladezeit (B) des Ladeimpulses (L), oder beide (C, B), zu erhöhen, wenn das Empfangssignal (20), vorzugsweise das abgeleitete Signal (tm), unter dem Zielwert (ts) oder einer unteren Grenze (tsmin) des Zielwertintervalls (tsmin, tsmax) liegt, und die Anzahl (C) von Erregungsimpulsen (S) und/ oder die Ladezeit (B) des Ladeimpulses (L) abzusenken, wenn das Empfangssignal (20), vorzugsweise das abgeleitete Signal (tm), über dem Zielwert (ts) oder einer oberen Grenze (tsmax) des Zielwertintervalls (tsmin, tsmax) liegt.

7. Detektor nach einem der vorhergehenden Ansprüche, **dadurch gekennzeichnet, dass** die Steuereinheit (30) ausgestaltet ist, die Anzahl (C) von Erregungsimpulsen (S) und/ oder die Ladezeit (B) des Ladeimpulses (L) mit einer jeweils vorbestimmten, oberen Grenze (Cmax, Bmax) zu vergleichen und nur zu erhöhen, wenn sie unterhalb ihrer jeweils vorbestimmten, oberen Grenze (Cmax, Bmax) liegt oder liegen, sodass eine obere Grenze der Sendeenergie nicht überschritten wird, und/ oder dass die Steuereinheit (30) ausgestaltet ist, die Anzahl (C) von Erregungsimpulsen (S) und/ oder die Ladezeit (B) des Ladeimpulses (L) mit einer jeweils vorbestimmten, unteren Grenze (Cmin, Bmin) zu vergleichen und nur abzusenken, wenn sie oberhalb ihrer jeweils vorbestimmten, unteren Grenze (Cmin, Bmin) liegt oder liegen, sodass eine untere Grenze der Sendeenergie nicht unterschritten wird.

8. Medizinisches Behandlungsgerät, insbesondere Dialysegerät (100), mit einem Detektor (1) gemäß einem der vorhergehenden Ansprüche, wobei ein von der Flüssigkeit durchströmbarer oder durchströmter Kanal (2) zwischen dem Ultraschall-Sender (4) und dem Ultraschall-Empfänger (8) angeordnet ist.

9. Verfahren zur Erkennung von Einschlüssen in einer extrakorporal strömenden Flüssigkeit, insbesondere in Blut oder in medizinischer Lösung, für einen Detektor auf Ultraschallbasis, insbesondere für einen Detektor (1) gemäß einem der Ansprüche 1 bis 7, mit Schritten:
- Ansteuern (S4) eines Ultraschall-Senders (4) mit einem Ansteuersignal (18), das eine Abfolge von Impulsen (L, S) umfasst, wobei die Abfolge einen Ladeimpuls (L) mit einer Ladezeit (B) und eine, auf den Ladeimpuls (L) folgende, Anzahl (C) von Erregungsimpulsen (S) umfasst, mittels einer Steuereinheit (30),
- Senden (S5) eines Ultraschalls (6) mit einer dem Ansteuersignal (18) gemäßen Sendeenergie durch einen die Flüssigkeit führenden Kanal (2), insbesondere einen Schlauch, mittels dem Ultraschall-Sender (4),
- Bereitstellen (S6) eines Empfangssignals (20) gemäß einer Empfangsenergie des Ultraschalls (6'), mittels einem Ultraschall-Empfänger (8),
- vorzugsweise Bilden (S7) eines vom Empfangssignal (20) abgeleiteten Signals (tm), insbesondere einer vom Empfangssignal (20) abgeleiteten statistischen Größe, und
- Anpassen (S8) der Sendeenergie, um das Empfangssignal (20), vorzugsweise das abgeleitete Signal (tm), auf einen Zielwert (ts) oder in ein Zielwert-Intervall (tsmin, tsmax) zu führen, mittels der Steuereinheit (30),
- **dadurch gekennzeichnet, dass** der Schritt Anpassen (S8) der Sendeenergie erfolgt durch einen Schritt Anpassen der Anzahl (C) von Erregungsimpulsen (S) und/ oder Anpassen der Ladezeit (B) des Ladeimpulses (L) des Ansteuersignals (18).

10. Verfahren nach Anspruch 9, **gekennzeichnet durch** Schritte:
- Einlesen (S1) wenigstens eines Parameters des Detektors (1), des Kanals (2) oder der Flüssigkeit, mittels der Steuereinheit (30),
- Aufrufen (S2) einer Korrelation, in der eine initiale Anzahl (C) von Erregungsimpulsen (S) und/ oder eine initiale Ladezeit (B) des Ladeimpulses (L), insbesondere eine initiale Sendeenergie, in Abhängigkeit des wenigstens einen Parameters abgelegt ist oder sind, mittels der Steuereinheit (30),
- Wählen (S3) der initialen Anzahl (C) von Erregungsimpulsen (S) und/ oder der initialen Ladezeit (B) des Ladeimpulses (L), und
- Ansteuern (S4) des Ultraschall-Senders (4) mit dem die initiale Anzahl (C) von Erregungsimpulsen (S) und/ oder die initiale Ladezeit (B) des Ladeimpulses (L) aufweisenden Ansteuersignal (18), um das Empfangssignal (20), vorzugsweise das davon abgeleitete Signal (tm), initial möglichst nahe an den Zielwert (ts) oder das Zielwert-Intervall (tsmin, tsmax) zu führen, mittels der Steuereinheit (30).

11. Verfahren nach Anspruch 9 oder 10, **dadurch gekennzeichnet, dass** der Schritt Anpassen (S8) der Anzahl (C) von Erregungsimpulsen (S) und/ oder der Ladezeit (B) des Ladeimpulses (L), insbesondere mit einer, vorbestimmten Schrittweite, solange erfolgt, bis das Empfangssignal (20), vorzugsweise das davon abgeleitete Signal (tm), auf den Zielwert (ts) oder in das Zielwertintervall (tsmin, tsmax) geführt ist, mittels der Steuereinheit (30).

12. Verfahren nach einem der Ansprüche 9 bis 11, **gekennzeichnet durch** Schritte:
- Erhöhen (S8.5) der Anzahl (C) von Erregungsimpulsen (S) und/ oder der Ladezeit (B) des Ladeimpulses (L), wenn das Empfangssignal (20), vorzugsweise das davon abgeleitete Signal (tm), unter dem Zielwert (ts) oder unter einer unteren Grenze (tsmin) des Zielwertintervalls (tsmin, tsmax) liegt, und
- Absenken (S8.6) der Anzahl (C) von Erregungsimpulsen (S) und/ oder der Ladezeit (B) des Ladeimpulses (L), wenn das Empfangssignal (20), vorzugsweise das davon abgeleitete Signal (tm), über dem Zielwert (ts) oder über einer oberen Grenze (tsmax) des Zielwertintervalls (tsmin, tsmax) liegt, mittels der Steuereinheit (30).

13. Verfahren nach Anspruch einem der Ansprüche 9 bis 12, **gekennzeichnet durch** Schritte:
- Vergleichen (S8.3) der Anzahl (C) von Erregungsimpulsen (S) und/ oder der Ladezeit (B) des Ladeimpulses (L) mit einer jeweils vorbestimmten, oberen Grenze (Cmax, Bmax), und
- Erhöhen (S8.5) der Anzahl (C) von Erregungsimpulsen (S) und/ oder der Ladezeit (B) des Ladeimpulses (L), nur, wenn sie unterhalb ihrer jeweils vorbestimmten, oberen Grenze (Cmax, Bmax) liegt oder liegen, mittels der Steuereinheit (30), sowie
- Vergleichen (S8.4) der Anzahl (C) von Erregungsimpulsen (S) und/ oder der Ladezeit (B) des Ladeimpulses (L) mit einer jeweils vorbestimmten, unteren Grenze (Cmin, Bmin), und
- Absenken (S8.6) der Anzahl (C) von Erregungsimpulsen (S) und/ oder der Ladezeit (B) des Ladeimpulses (L), nur, wenn sie oberhalb ihrer jeweils vorbestimmten, unteren Grenze (Cmin, Bmin) liegt oder liegen, mittels der Steuereinheit (30).

14. Verfahren nach Anspruch 13, **gekennzeichnet durch** wenigstens einen der Schritte:
- Abfragen (S8.71), ob die Anpassung der Sendeenergie auf Basis der Anpassung der Ladezeit (B) des Ladeimpulses (L) fortgeführt werden soll, wenn das Vergleichen (S8.3) der Anzahl (C) von Erregungsimpulsen (S) mit ihrer vorbestimmten, oberen Grenze (Cmax) ergibt, dass die Anzahl (C) von Erregungsimpulsen (S) oberhalb ihrer vorbestimmten, oberen Grenze (Cmax) liegt, oder wenn das Vergleichen (S8.4) der Anzahl (C) von Erregungsimpulsen (S) mit ihrer vorbestimmten, unteren Grenze (Cmin) ergibt, dass die Anzahl (C) von Erregungsimpulsen (S) unterhalb ihrer vorbestimmten, unteren Grenze (Cmin) liegt;
- Abfragen (S8.72), ob die Anpassung der Sendeenergie auf Basis der Anpassung der Anzahl (C) von Erregungsimpulsen (S) fortgeführt werden soll, wenn das Vergleichen (S8.3) der Ladezeit (B) des Ladeimpulses (L) mit ihrer vorbestimmten, oberen Grenze (Bmax) ergibt, dass die Ladezeit (B) des Ladeimpulses (L) oberhalb ihrer vorbestimmten, oberen Grenze (Bmax) liegt, oder wenn das Vergleichen (S8.4) der Ladezeit (B) des Ladeimpulses (L) mit ihrer vorbestimmten, unteren Grenze (Bmin) ergibt, dass die Ladezeit (B) des Ladeimpulses (L) unterhalb ihrer vorbestimmten, unteren Grenze (Bmin) liegt.

15. Computerlesbares Speichermedium, das Befehle umfasst, die bei einer Ausführung durch einen Computer diesen veranlassen, die Verfahrensschritte des Verfahrens für den Detektor (1) gemäß einem der Ansprüche 9 bis 14 auszuführen.
